# EUROPEAN PATENT APPLICATION

(11) **EP 1 829 522 A1**
(43) Date of publication of application: **05.09.2007**
(21) Application number: 06024820.0
(22) Date of filing: 30.11.2006
(51) Int. Cl.: A61K 8/19, A61Q 1/00, A61K 8/58

(54) **Cosmetic compositions comprising sub-micron boron nitride particles**

(30) Priority: 01.03.2006 US 777917 P; 28.10.2006 US 554004
(71) Applicant: General Electric Company, Schenectady, NY 12345 (US)
(72) Inventor: Butts, Matthew David, Rexford New York 12148 (US); Sinha, Moitreyee, Niskayuna New York 12309 (US); Genovese, Sarah Elizabeth, Delmar New York 12054 (US); Yamada, Masako, Niskayuna New York 12309 (US)
(74) Representative: Zimmermann, Gerd Heinrich

(57) **Abstract**

A cosmetic composition for use on hair, skin or nails, containing a safe and effective amount of boron nitride powder has been found which improves the appearance of skin by blurring lines and wrinkles while at the same time providing coverage of age spots, blemishes and other discolorations.

## Description

The present invention relates to compositions comprising sub-micron Boron Nitride particles that manipulate light. The invention also relates to a method to optimize the optical diffusion effect of light, consequently, blurring the appearance of wrinkles, and lines as well as lessening the visibility of discolorations to enhance skin appearance.

Makeup and foundation are typically used to enhance one's appearance, including reducing the appearance of wrinkles or flaws in one's skin. Cosmetic compositions in the prior art typically contain components such as metallic oxides to confer an opacity to the composition. These components may be excellent for evening out skin tone, but may not be very flattering to certain types of skin. Furthermore, in their tendency to accumulate in furrows, these components may actually serve to emphasize the deeper wrinkles and flaws rather than hide them.

In the prior art, one attempt to hide skin flaws is the use of "soft focus" types of powders in cosmetic compositions. These materials are spherical powders that are known in the cosmetic industry for their light-scattering properties on the skin. U.S. Patent No. 5,223,559 discloses a cosmetic composition capable of blurring skin defects containing spherical or spheroidal filler particles 0.5 to 50 µm in size. Such powders, for example, spherical silicas, polyethylene, or polymethylmethacrylate ("PMMA"), operate on the principle of diffusing light incident on the face in such a way that the overall appearance of the skin is somewhat blurred in the viewer's eye, thereby minimizing the viewer's ability to detect lines and wrinkles on the skin. However there is typically a trade-off between the ability to blur lines and hide color blemishes. A common deficiency in these formulations is their ability to simultaneously provide the lateral diffusion of light to blur topographical defects such as wrinkles and the opacity to cover color blemishes.

It has been shown in a 2004 proceeding of the IFSCC (International Federation of Society of Cosmetic Chemists) that lowering the visibility of defects or blurring the lines / wrinkles below acceptable thresholds is one of the key criteria for the selection of materials for cosmetic compositions. U.S. Patent No. 6,511,672 discloses compositions containing pigments which optimize the optical diffusion effect of light, specifically, a first platelet of alumina treated with a metal oxide in combination with at least one second platelet treated with at least one spherical scattering component. Together the pigments and the platelets produce a sum of reflected light off of the skin that appears gentle and soft and blurs the flaws of the skin. Ip.com Publication No. 000011624 discloses the use of Tospearl^{®} (a silicone microsphere) in cosmetic compositions to provide soft focus effects in personal care applications, again working on the same principle of blurring effects through highly diffused transmission and highly scattered reflectance.

The use of boron nitride fillers in cosmetic compositions including hair and nail treatments is not new, as they have been previously used in the prior art, primarily in very small quantities as particulate matters in amounts ranging from 0.1 - 70 wt.-% depending on the type of cosmetic compositions, i.e., eyeshadows, lipsticks, foundation make-up, powder, blushes, shampoos, conditioners, etc. BN fillers have been used with a host of other fillers in the prior art cosmetic compositions, such as fumed silica, spherical silica, polymethylmethacrylate, micronized teflon, acrylate polymers, aluminum silicate, aluminum starch octenylsuccinate, bentonite, calcium silicate, cellulose, chalk, corn starch, rice starch, silica, talc, mica, titanium dioxide, soy flour, tin oxide, titanium hydroxide, walnut shell powder, etc. or mixtures thereof. BN and other fillers in the prior art may be surface treated with lecithin, amino acids, mineral oil, silicone oil, or various other agents either alone or in combination, which coat the powder surface and render the particles hydrophobic in nature. They have been used in cosmetic compositions for various purposes, ranging from providing protection from sun exposure, reducing the shine from oily skin, providing a cooling effect (due to its thermal conductivity), to providing a smoothness effect, etc., to the wearer of the cosmetic compositions. The BN filler for use in the composition of the prior art is of a size of greater than 5 microns.

JP Patent Publication No. 2003-104841 discloses cosmetic compositions with spherical BN particles having an average diameter of 1 to 20 µm. The collapsible nature of the spherical BN particles allows the filler to give the cosmetic composition an excellent luster.

There continues to be a need for a cosmetic composition that can reduce the appearance of lines and wrinkles on skin and provide opacifying properties to cover blemishes, or in some instances, that can improve the appearance of hair, and / or nails. The present invention now provides compositions which minimize and / or hide surface flaws, e.g., wrinkles and blemishes.

The present invention intends to overcome at least some of the above problems. The object is solved by the composition according to independent claims 1 and 2, the method for manufacturing the composition of the present invention according to independent claim 9, the use of the compositions according to independent claim 10 and a method for improving the appearance of skin according to independent claim 11.

Further advantages, features, aspects and details of the invention are evident from the dependent claims and the description. The claims are intended to be understood as a first non-limiting approach of defining the invention in general terms.

The present invention generally relates to a cosmetic composition for enhancing the appearance of skin, hair, or nails.

In a further aspect the invention relates to a composition comprising a sufficient amount of boron nitride powder containing platelets having an average primary particle size of less than 1 micron, for the composition to have a total transmission of less than 60% and an MTR of less than 600.

In a further aspect the present invention relates to a cosmetic composition comprising a sufficient amount of boron nitride powder containing platelets having an average primary particle size of less than 1 micron, for the composition to have an MTR of 600 or less at 50% transmission.

In another aspect the present invention relates to the use of the composition for enhancing the appearance of skin, hair, or nails,

In one aspect of the invention, a method for reducing the appearance of defects on hair, skin, and nails, e.g., lines, wrinkles, and scars on skin, is disclosed. The method comprises applying a composition containing sub-micron boron nitride powder having an average particle size of less than 1 micron. In one example, the composition has the effect of blurring lines, scars, and / or wrinkles on the skin while providing opacity to hide color blemishes.

In a second aspect of the invention for a foundation formulation, a synergistic amount of light-diffusing particles such as silicone microspheres is added to the composition to further achieve a desired combination of transmission as well as line blurring on the skin.

The invention further relates to cosmetic compositions that can blur skin defects, with the composition containing a safe and effective amount of sub-micron boron nitride powder having an average particle size of less than 1 micron.

Thus, a cosmetic composition for use on hair, skin or nails, containing a safe and effective amount of boron nitride powder has been found which improves the appearance of skin by blurring lines and wrinkles while at the same time providing coverage of age spots, blemishes and other discolorations.

As used herein, approximating language may be applied to modify any quantitative representation that may vary without resulting in a change in the basic function to which it is related. Accordingly, a value modified by a term or terms, such as "about" and "substantially," may not be limited to the precise value specified, in some cases.

The term "safe and effective amount" as used herein means an amount of a compound, component, or composition sufficient to significantly induce a positive benefit, i.e., a positive cosmetic appearance or feel benefit such as reducing the appearance of lines and wrinkles on the skin, but low enough to avoid serious side effects, i.e. to provide a reasonable benefit to risk ratio, within the scope of sound medical judgement.

All percentages and ratios used herein are by weight of the total composition and all measurements made are at 25 °C, unless otherwise designated. Unless otherwise indicated all percentages, ratios and levels of ingredients referred to herein are based on the actual amount of the ingredient, and do not include solvent, fillers or other materials which may be combined with the ingredient in commercially available products.

Active and other ingredients useful herein may be categorized or described herein by their cosmetic and/or therapeutic benefit or their postulated mode of action. However, it is to be understood that the active and other ingredients useful herein can in some instances provide more than one cosmetic and/or therapeutic benefit or operate via more than one mode of action. Therefore, classifications herein are made for the sake of convenience and are not intended to limit an ingredient to the particularly stated application or applications listed.

As known in the art, there is a link between the optics of a defect and its effect on human perception as quantified by certain optical parameters such reflected light, color of the surface, % transmission, etc. For cosmetic compositions, coverage refers to the opacity of the formulation. Foundations typically have higher coverage to hide age spots, blemishes etc. In order to do this, some opacity is desired. A measure of opacity is the total amount of transmitted light (i.e., how opaque or transparent) measured using McBeth ColorEye with a range from 0 to 100 %. At 0 %, the formulation is completely opaque like chalk. At 100 %, the formulation is completely transparent like glass.

Other than providing coverage, a cosmetic formulation should also hide wrinkles. One way to measure line-blurring or quantify the modulation of light, and thus the loss of image formation or the blurring of lines is through the measurement of the modulation transfer ratio (MTR) or modulation transfer function (MTF). The modulation transfer function (MTF) is a graphical description of the blur, or resolution characteristics, using an imaging system or its individual components. An MTF test object has peaks and valleys, with one peak and valley make up 1 cycle of the object. When light passes through the MTF test object, the camera imaging system measures the contrasts created by the features on the test object. When a film with the formulation drawn on it is inserted between the MTF test object and the camera imaging system, it will blur out the features with various spatial frequencies. The ability of a formulation to blur out the lines with different spatial frequencies is related to the way the particles scatter light present.

In the MTR test, the higher the modulation ratio, the higher the contrast. Thus, the lower the contrast, the higher is the loss of image information or the blurring of lines. In Human Vision and Optical Imaging literature, the MTF or MTR are commonly used techniques to quantify human perception of line-blurring. MTR is a ratio calculated from the intensity profile (ratio of max and min intensity), i.e., a measure of how blurred the profile is. The "mean" is the average intensity of the profile, or a measure of how much light is transmitted through.

The use of BN as a filler in cosmetic compositions provides all the advantages recognized in the prior art, i.e., cooling effect, smooth appearance, reducing shine, etc. However, Applicants have found that the use of certain BN powder, specifically, submicron powder, in a formulation such as a foundation, surprisingly creates the illusion of substantially flawless skin, by blurring the appearance of wrinkles and lines on the wearer's skin while also providing noticeable coverage of color blemishes, spots and defects.

Sub-micron boron nitride fillers: As used herein, sub-micron boron nitride refers to boron nitride ("BN") powder having an average particle size of less than 1 micron (1000 nm). Sub-micron BN particles can be made using various methods known in the art. In one process, by combining chemical vapor deposition and pyrolysis of trimethoxyborane under ammonia atmosphere, spherical boron nitride particles with a uniform diameter distribution from 50 to 400 nm can be synthesized. Under high-resolution transmission electron microscopy, the sub-micron boron nitride particles made by this method exhibit a slightly distorted arrangement of the shell layers.

In a second process, sub-micron BN filler can be prepared by breaking agglomerates of commercially available BN powder (with sizes greater than 1 micron) through sonication of a liquid suspension of BN in water and surfactant in an ultrasonic bath. Sub-micron boron nitride powders are commercially available from a number of sources, including General Electric Company of Strongsville, OH, or St. Gobain Ceramics of Worcester, MA.

In one embodiment, the sub-micron BN powder has an average particle size in the range of 0.10 (100 nm) to 0.8 µm (800 nm). In a second embodiment, the BN powder has an average particle size of 200 nm to 700 nm. In a third embodiment, the BN powder has an average particle size of 200 to 600 nm. In a fourth embodiment, the BN powder has an average particle size of 200 to 500 nm.

In one embodiment, the sub-micron BN powder is present in the composition as a dispersion in a safe and effective amount, such that when applied onto the skin, the composition effectively blurs the appearance of lines and / or wrinkles on the skin to make the skin appear without lines and / or wrinkles while at the same time providing noticeable coverage. In one embodiment of the invention, a sufficient amount of sub-micron BN is used for line blurring benefit indicated by an MTR of 600 or less at 50 % transmission, and with the coverage benefit described as having total transmission of 60% or lower. In a second embodiment, the sub-micron BN is present in a sufficient amount for the composition to have an MTR of 500 or less at 50 % transmission. In a third embodiment, the sub-micron BN is present in a sufficient amount for the composition to have a total transmission of less than 60 %. In a fourth embodiment, the sub-micron BN is present in a sufficient amount for the composition to have a total transmission of less than 60 % (McBeth ColorEye test) and an MTR of less than 600.

Coverage refers to how opaque the formulation is. Foundations typically have higher coverage to hide age spots, blemishes etc. A measure of coverage is the degree of transmission (i.e. how opaque or transparent). % transmission ranges from 0 to 100% - 0 means totally opaque like chalk, 100% means totally transparent. In order for a formulation to provide coverage, it is desired to have transmission lower than 60 %. So for BN to simultaneously provide coverage and line-blurring effect, it should have total transmission lower than 60 % (measure of coverage) and MTR lower than 600 (measure of line-blurring).

In one embodiment, the BN powder is present in a safe and effective amount ranging from 0.1 to about 70 wt.-%, in a composition otherwise comprising 0 to 90 wt. % water, 0 to 10 wt. % surfactant, and 0 to 40 wt. % of other known active ingredients or additives in the art. In a second embodiment, the BN powder is present in a safe and effective amount ranging from 0.1 to about 70 wt. %, in a composition otherwise comprising 0 to 90 wt. % silicone and 0 to 40 wt. % of other known active ingredients or additives in the art. In a third embodiment, this amount ranges from 0.5 to 30 wt. -%. In a fourth embodiment, from 1 to 20 wt.-%.

Optional Synergistic Light Diffusing Component: In one embodiment, the sub-micron BN may be used in conjunction with known light-diffusing particles in the prior art to optimize the loss of image formation or line-blurring effects as a cosmetic application. Examples of light-diffusing particles include microspheres and microsphere complexes containing polymethyl methacrylate, polyethylene, silicon resin, silica, etc. The selection of the synergistic light-diffusing component for use with the sub-micron boron nitride powder depends on the refractive index of the matrix to optimize the scattering of light and specific end-use applications, as each microsphere has its own feel from smooth / wet, soft, creamy, hard to dry, etc.

In one embodiment, light-diffusing particles such as silicone microspheres, e.g., microspheres comprising alkyl phenyl silsesquioxane, polymethylsilsesquioxane, etc. are used in conjunction with the sub-micron boron nitride powder. Such use is advantageous in the sense that the sub-micron BN powder is an opacifier, thus a combination with a transparent component in a silicone matrix such as silicone microspheres can be used to further improve the appearance of skin, nails, or hair. In one embodiment the ratio of BN to light-diffusing particles microspheres is optimized to achieve a desired combination of transmission as well as line blurring.

In one embodiment, silicone microspheres sold under the trade name of Tospearl^{®} from GE Toshiba Silicones are added in a sufficient amount that does not impair the coverage or blurring effects of the cosmetic composition. Tospearl (INCI name Polymethylsilsesquioxane) is a silicone microsphere made by the hydrolysis and condensation of methyltrimethoxysilane, available in different average primary particle sizes ranging from 0.5 to 12 microns.

In one embodiment, Tospearl microspheres having a narrowly controlled average particle size in the range of 0.3 to 0.9 microns are used in the composition of the invention. In a second embodiment, Tospearl microspheres having average primary particle sizes in the range of 0.4 to 0.8 microns are used. In a third embodiment, Tospearl microspheres having average primary particle sizes in the range of 0.5 to 0.7 microns are used.

In one embodiment, Tospearl microspheres are added to the sub-boron nitride powder in a safe and effective amount of 0.1 to about 30 wt.-%. In a second embodiment, this amount ranges from 0.5 to 20 wt.-%. In a third embodiment, from 1 to 10 wt.-%.

Optional Nano-Particle Fillers. In one embodiment of the invention, the sub-micron BN fillers may be used in conjunction with nano-pigments, e.g., pigment in which the mean size of the unit particles range from 0.01 to 0.15 microns, in an amount ranging from 0.1 to 30 wt. %. As used herein, nano-pigments refer to pigment fillers wherein the particles are substantially spherical such as nano-particles of iron oxide, titanium dioxide, chromium hydroxide, calcium silicate, chromium oxide, kaolin, manganese violet, carbon black, bismuth oxychloride, and mixtures thereof. The optional use of nano-particles helps form a barrier to UV radiation.

Other Components: The composition also can comprise other components that may be chosen depending on the carrier and/or the intended use of the formulation.

In one embodiment, the composition comprises a liquid binder chosen from water, water-miscible organic solvents, oils, and mixtures thereof; for example, the liquid binder may be chosen from water and water-miscible organic solvents, and mixtures thereof.

Additional components include, but are not limited to, water soluble sunscreens (such as Eusolex 232); oil soluble sunscreens (such as octyl methoxycinnamate); and organic sunscreens (such as camphor derivatives, cinnamates, salicylates, benzophenones, triazines, PABA derivatives, diphenylacrylate derivatives, and dibenzoylmethane derivatives); antioxidants (such as BHT); chelating agents (such as disodium EDTA); emulsion stabilizers (such as carbomer); preservatives (such as methyl paraben); fragrances (such as pinene); flavoring agents (such as sorbitol); humectants (such as glycerine); waterproofing agents (such as PVP/Eicosene copolymer); water soluble film-formers (such as hydroxypropyl methylcellulose); oil-soluble film formers (such as hydrogenated C-9 Resin); moisturizing agents (such as cholesterol); cationic polymers (such as polyquaternium 10); anionic polymers (such as xanthan gum); pigment wetting agents (such as Arlacel^{™} P100, Emerest^{™} 2452); vitamins (such as tocopherol); and the like.

In one embodiment, the composition can also encompass one or more active components, and as such can be either cosmetic or pharmaceutical compositions. Examples of useful actives include, but are not limited to, those that improve or eradicate age spots, keratoses and wrinkles, analgesics, anesthetics, anti-acne agents, antibacterials, antiyeast agents, antifungal agents, antiviral agents, antidandruff agents, antidermatitis agents, antipruritic agents, antiemetics, antimotion sickness agents, antiinflammatory agents, antihyperkeratolytic agents, anti-dry skin agents, antiperspirants, antipsoriatic agents, antiseborrheic agents, hair conditioners and hair treatment agents, antiaging agents, antiwrinkle agents, antiasthmatic agents and bronchodilators, sunscreen agents, antihistamine agents, skin lightening agents, depigmenting agents, wound-healing agents, vitamins, corticosteroids, tanning agents, sunscreens or hormones.

More specific examples of useful active agents include retinoids such as retinol, and esters, acids, and aldehydes thereof; ascorbic acid, and esters and metal salts thereof, tocopherol and esters and amide derivatives thereof; shark cartilage; milk proteins; alpha- or beta-hydroxy acids; DHEA and derivatives thereof; topical cardiovascular agents; clotrimazole, ketoconazole, miconozole, griseofulvin, hydroxyzine, diphenhydramine, pramoxine, lidocaine, procaine, mepivacaine, monobenzone, erythromycin, tetracycline, clindamycin, meclocyline, hydroquinone, minocycline, naproxen, ibuprofen, theophylline, cromolyn, albuterol, hydrocortisone, hydrocortisone 21-acetate, hydrocortisone 17-valerate, hydrocortisone 17-butyrate, betamethasone valerate, betamethasone diproprionate, triaminolone acetonide, fluocinonide, clobetasol, proprionate, benzoyl peroxide, crotamiton, propranolol, promethazine, and mixtures thereof.

In one embodiment, the composition also contains colorant reflective particles other than those comprising mica substrates, e.g., particles comprising a synthetic substrate, other than mica, coated at least partially with at least one layer of at least one metal compound, and for example, a metal oxide. In yet another embodiment, the composition further comprises particles such as surface treated zinc oxide and titania particles, particularly nano-particles.

In one embodiment of the invention, the composition can also contain optional inorganic powder as fillers, e.g., silica, polymethylmethacrylate, calcium silicate, cellulose, chalk, corn starch, rice starch, talc, mica, bismuth oxychloride, barium sulfate, mica, sericite, muscovite, synthetic mica, titanium oxide coated mica, titanium oxide coated bismuth oxychloride, titanium oxide coated talc, platelet iron oxides, metal powders such as aluminum, lauryl lysine and platelet talc, etc. Although the powder can be any of the type ordinarily used in cosmetics, in one embodiment, the powder can be a non-matte powder in an amount of about 2 to about 10 %. In a second embodiment, the optional powder filler has an average particle size of about 2 to 50 µm.

Applications of the Composition of the Invention: The sub-micron BN blends of the present invention can be used in any type of skin treatment or cosmetic product, hair treatment product, as well as nail treatment products. Examples of skin treatment products include, but are not limited to, lip products, moisturizers, anti-aging products, lifting treatments, cellulite treatments and eye treatments, etc. The cosmetic or makeup products of the invention include, but are not limited to, liquid foundations, blushes, pressed or loose powders, concealers, bronzers, eyeshadows, concealer, nail enamels, a make-up rouge, a body make-up product, eyeliners, lipsticks, and lip glosses. The products of the invention can take any form which is typical of cosmetic products, for example, hot pour formulations, water-in-oil emulsions, oil-in-water emulsions, gels, sticks, sprays, anhydrous formulations, and pressed or loose powders. There is no limitation on the type of vehicle that can be employed. In particular, the preferred identity of the vehicle will be largely controlled by the type of product into which the components are to be incorporated. In one embodiment, the composition is an oil-in-water emulsion. In a second embodiment, it is a silicone-in-water emulsion. In a third embodiment, it is a water-in-silicone oil emulsion.

The composition of the invention is used in the same manner as any typical foundation or make-up composition. In one example, the user applies the formulation with, for example, the fingers or an appropriate applicator to the skin on which the lines to be disguised appear.

The invention is further illustrated by the following non-limiting examples:

Example 1: BN powder having an average primary particle size of 243 nm is compared with TiO₂ having an average particle size of 170 nm. TiO₂ is a filler used in cosmetic compositions of the prior art. Simple formulations for foundations were prepared such that both formulations had equal opacity or ability to hide color blemishes. MTR measurements are made comparing compositions containing submicron BN particles OR TiO₂ per formulations below. In a third formulation, the BN amount was reduced ½ and replaced with Tospearl 3210, a silicone microsphere from GE Toshiba Silicones of Japan.

In the formulation, the following ingredients were used: 10% particles; 10% SF1528 (GE Silicones, Waterford, NY); 22.8% 600Mcstk PDMS; 57.2 % D5. The formulations were drawn out forming films of 25 microns in wet thickness. In transmission tests, both films have comparable transmission of ~ 50 %. The results indicate: a) MTR of 3766 (with a mean of 1537) for a mask / film with no additive; b) MTR of 550 (with a mean of 356) for the formulation containing TiO₂; c) MTR of 6 (with a mean of 349) for the formulation containing the sub-micron BN; d) MTR of 580 (with a mean of 377) for the formulation containing BN and Tospearl 3120.

MTR and mean values are in pixel units. The "mean" intensity refers to the total transmitted light which is the spatial average over the measurement area. Since both these films have 50% total transmission, the mean values are similar. MTR values reflect the blurring of lines with lower numbers representing higher degree of blurring.

Example 2: Example of a moisturizer formulation

| Phase | Ingredients | wt.-% |
|---|---|---|
| A | Deionized Water | 83.07 |
| | Versene NA-2 | 0.05 |
| | Carbopol Ultrez 10 | 0.2 |
| | Phenonip | 0.4 |
| B | Boron Nitride OR TiO₂ | 1 |
| | Butylene Glycol USP | 2.5 |
| C | Schercemol CO | 5 |
| | Arlacel 60 | 1.5 |
| | Tween 60 | 1 |
| | Lipowax D | 1 |
| | SF1214 | 1.5 |
| | Phenonip | .6 |
| D | Deionized Water | I |
| | AMP-95 | 18 |
| F | Sepiplus 400 | 1 |

Example 2: This example illustrates a concealer formulation:

| | Ingredients | Wt.-% |
|---|---|---|
| A | Water (q.s. to 100%) | 53.50 |
| | Magnesium Aluminum Silicate (Veegum) | 2.00 |
| B | Propylene Glycol | 8.00 |
| | Triethanolamine (TEA 99%) | 1.50 |
| | Cellulose Gum (CMC-7LF) | 1.00 |
| | Preservatives (water soluble) | q.s. |
| C | Boron nitride having an average particle size of 243 nm | 12.00 |
| | Iron Oxides (C33-210 Cosmetic Yellow) | 1.00 |
| | Iron Oxides (C33-215 Cosmetic Russet) | 0.50 |
| | Iron Oxides (C33-225 Cosmetic Brown) | 0.50 |
| D | Mearlmica CF | 6.00 |
| | Boron nitride having an average particle size of 243 nm | 5.00 |
| E | Stearic Acid (Emersol 132) | 3.00 |
| | Glyceryl Stearate SE (Cerasynt Q) | 2.00 |
| | Mineral Oil (Carnation White Mineral Oil) | 2.00 |
| | Isopropyl Lanolate (Amerlate P) | 1.50 |
| | Isostearic Acid (Emersol 871) | 0.50 |
| | Preservatives (oil soluble) | q.s. |
| | | 100.00 |

In this example, Veegum is first dispersed into water, using high shear mixing until smooth. Next, Phase B slurry is added to Phase A and is mixed until smooth. Phase C is pulverized and added to Phase A-B, using high shear mixing until smooth. Phase D is added to Phase A-B-C, while heating to 75 +/- 5°C, and is mixed until smooth. In a support vessel, Phase E ingredients are heated to 75 +/- 5°C with gentle agitation.

Phase E is added to Phase A-B-C-D with gentle agitation, while maintaining temperature at 75 +/- 5°C. In the next stage, constant agitation is maintained and the batch is cooled to 35 +/- 5°C. Concealer batch is stored and filled into appropriate containers.

Example 3: This example illustrates a liquid foundation formulation:

| Phase | Ingredients | wt.-% |
|---|---|---|
| A | Candelilla Wax | 2.75 |
| | Carnauba Wax | 1.25 |
| | Beeswax | 1.00 |
| | Ceresine Wax | 5.90 |
| | Ozokerite Wax | 6.75 |
| | Microcrystalline Wax (Multiwax 180W) | 1.40 |
| | Oleyl Alcohol (Novol) | 3.00 |
| | Isostearyl Palmitate (Jeechem ISP) | 7.50 |
| | Isostearyl Isostearate (Schercemol 1818) | 5.00 |
| | Caprylic/Capric Triglyceride (Neobee M-5) | 5.00 |
| | Bis-Diglycerylpolyalcohol Adipate-2 (Softisan 649) | 2.00 |
| | Acetylated Lanolin Alcohol (Acetulan) | 2.50 |
| | Sorbitan Tristearate (Crill 35) | 2.00 |
| | Aloe Vera (Veragel Lipoid 1:1) | 1.00 |
| | Castor Oil (q.s. to 100%) | 34.75 |
| | Boron nitride having an average particle size of 243 nm | 3.00 |
| | Tocopheryl Acetate | 0.20 |
| | Antioxidant & Preservatives | q.s. |
| B | Duocrome Sparkle RB 624J | 3.00 |
| | Cellini Coral (420CR40F) | 12.00 |
| C | Fragrance | q.s. |
| | | 100.00 |

First, all Phase A ingredients are weighed in a heated vessel and temperature is raised to 85 +/- 3°C, stirring until melted and uniform. Phase B is added to Phase A, and is mixed until melted and uniform. Phase C is then added to Phase A - B, and is mixed with constant stirring. The mixture is then poured at 75 +/- 5°C into molds and cooled.

Example 4: This example illustrates a loose face powder formulation:

| Phase | Ingredients | wt.-% |
|---|---|---|
| A | Mearlcite SRA (q.s. to 100%) | 48.40 |
| | Mearlmica CF | 29.30 |
| | Zinc Stearate | 3.00 |
| | Silk Powder (Crosilk Powder) | 2.00 |
| | Magnesium Carbonate (Light USP 309) | 2.00 |
| | Boron nitride having an average particle size of 243 nm | 10.00 |
| | Iron Oxides (C33-210 Cosmetic Yellow) | 0.40 |
| | Iron Oxides (C33-215 Cosmetic Russet) | 0.07 |
| | Iron Oxides (C33-5201 Cosmetic Jet Black) | 0.10 |
| | D&C Red No. 30 Lake (C37-5290 Permanent Pink) | 0.23 |
| | Preservatives | q.s. |
| B | Caprylic/Capric Triglyceride (Neobee M-5) | 2.25 |
| | Phenyl Trimethicone (Dow Coming 556 Fluid) | 1.75 |
| | Tocopheryl Acetate | 0.50 |
| | Fragrance | q.s. |
| | | 100.00 |

In this example, Phase A is first thoroughly blended and dispersed in appropriate dry blending/dispersing equipment. Next, Phase B ingredients are blended until uniform. Phase B is sprayed into pre-mixed Phase A and blended with the mixture. Lastly, the Phase A-B mixture is pulverized and stored in appropriate containers.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

This application claims the benefits of U.S. 60/777,917 filed March 1, 2006 which patent application is fully incorporated herein by reference.

All citations referred herein are expressly incorporated herein by reference.

## Claims

1. Cosmetic composition for enhancing the appearance of skin, hair, or nails, the composition comprising a sufficient amount of boron nitride powder containing platelets having an average primary particle size of less than 1 micron, for the composition to have a total transmission of less than 60% and an MTR of less than 600.

2. Cosmetic composition for enhancing the appearance of skin, hair, or nails, the composition comprising a sufficient amount of boron nitride powder containing platelets having an average primary particle size of less than 1 micron, for the composition to have an MTR of 600 or less at 50% transmission.

3. The composition of claim 1 or 2, the composition comprising a sufficient amount of boron nitride powder containing platelets having an average primary particle size of 100 nm to 800 nm, for the composition to have an MTR of 500 or less at 50% transmission.

4. The composition of any of claims 1 - 3, wherein the composition comprises from 0.1 to about 70 wt. % of boron nitride powder containing platelets having an average primary particle size of 100 nm to 800 nm.

5. The composition of any of claims 1 - 4, wherein the composition comprises from 1 to about 20 wt. % of a sub-micron boron nitride powder containing platelets having an average primary particle size of 200 nm to 500 nm.

6. The composition of any of claims 1 - 5, wherein the composition further comprises from 1 to about 20 wt.-% amount of polymethylsilsesquioxane microspheres having an average primary particle size ranging from 0.5 to 1 micron.

7. The composition of any of claims 1 - 6, wherein the composition further comprises from 1 to about 20 wt.-% of nano-pigments selected from the group consisting of iron oxide, titanium dioxide, chromium hydroxide, calcium silicate, chromium oxide, kaolin, manganese violet, carbon black, bismuth oxychloride, and mixtures thereof.

8. The composition of any of claims 1 - 7, wherein the composition is one of a solid form, a semi-solid form, a pressed powder, and loose powder, for use as one of a lipstick, blush, concealer, nail enamel, eye shadow, a foundation, a make-up rouge, and a body make-up product.

9. Method for manufacturing a composition according to claim 1 - 7, chracterized in that the components are if necessary successively mixed together.

10. Use of a composition according to one of claim 1 to 9

11. A method for improving the appearance of skin, hair, or nails, wherein the method comprises applying to skin, hair or nails a composition having an MTR of 500 or less at 50 % transmission and a safe and effective amount of boron nitride powder containing platelets having an average primary particle size of less than 1 micron.

12. The method of claim 11, wherein the composition further comprises a safe and effective amount of silicone microspheres having an average primary particle size ranging from 0.3 to 15 microns.

13. The method of claim 11 or 12, wherein the sub-micron boron nitride powder contain platelets having an average primary particle size of 100 nm to 800 nm.

14. The method of any of claims 11-13, wherein the composition comprises from 0.1 to about 70 wt. % of a sub-micron boron nitride powder containing platelets having an average primary particle size of 100 nm to 800 nm and from 1 to about 20 wt.-% of silicone microspheres having an average primary particle size ranging from 0.5 to 15 microns.
